**Europäisches Patentamt**

(19) **European Patent Office**

**Office européen des brevets**

(11) Veröffentlichungsnummer: **0 035 125**
**B1**

(12) ## EUROPÄISCHE PATENTSCHRIFT

(45) Veröffentlichungstag der Patentschrift:
28.03.84

(51) Int. Cl.³: **C 12 P 1/00**, A 23 K 1/17 //
(C12P1/00, C12R1/00)

(21) Anmeldenummer: 81100801.0

(22) Anmeldetag: 05.02.81

(54) **Feststoff der Salinomycin-Kulturbrühe und Verfahren zu seiner Gewinnung.**

(30) Priorität: 15.02.80 DE 3005642

(43) Veröffentlichungstag der Anmeldung:
09.09.81 Patentblatt 81/36

(45) Bekanntmachung des Hinweises auf die Patenterteilung:
28.03.84 Patentblatt 84/13

(84) Benannte Vertragsstaaten:
AT BE CH DE FR GB IT LI NL SE

(56) Entgegenhaltungen:
EP - A - 0 003 644
CH - A - 251 112
DE - A - 2 035 814
DE - A - 2 154 633
GB - A - 1 378 414

CHEMICAL ABSTRACTS, Band 90, Nr. 19, 7. Mai 1979,
Seite 462, Nr. 150298a Columbus, Ohio, U.S.A.

(73) Patentinhaber: **HOECHST AKTIENGESELLSCHAFT,
Postfach 80 03 20, D-6230 Frankfurt am Main 80 (DE)**

(72) Erfinder: **Höhl, Rolf, Dr., Am Wickerbach 20,
D-6238 Hofheim am Taunus (DE)**
Erfinder: **Heine, Helmut, Kirchgasse 12,
D-6242 Kronberg/Taunus (DE)**

Feststoff der Salinomycin-Kulturbrühe und Verfahren zu seiner Gewinnung

Es ist bekannt, dass Salinomycin al als Futterzusatz sehr gute Wirkungen auf Wachstum und Futterverwertung, beispielsweise bei Wiederkäuern und Schweinen zeigt. Auch als Coccidiostatikum findet es in der Tiermedizin Verwendung.

Die Isolierung des Wirkstoffs aus der Fermentationsbrühe ist, da zahlreiche mit hohen Investitions-, Material- und Energiekosten belastete Stufen, wie z.B. Lyophilisation oder Extraktion durchlaufen werden müssen, sehr aufwendig und kostspielig.

Für die Verwendung von Salinomycin als Futterzusatz ist es jedoch nicht erforderlich, den Wirkstoff rein darzustellen. Er kann für diesen Zweck zusammen mit dem Feststoff aus der Salinomycin-Kulturbrühe gewonnen und in dieser Form eingesetzt werden. Das so erhaltene Produkt zeichnet sich durch erheblich niedrigere Herstellungskosten aus.

Bei der Isolierung des Feststoffs aus der Kulturbrühe traten bei dem Versuch, die in vergleichbaren Fällen gut durchführbare Zerstäubungstrocknung anzuwenden, unerwartete Schwierigkeiten auf, da der Feststoff verbackte und Klumpen bildete, die sich nicht weiter verarbeiten liessen. Die Verbackungen und Klumpenbildungen werden offensichtlich durch die Stoffeigenschaften des Salinomycins und auch durch im Feststoff enthaltene Fette und fettähnliche Produkte hervorgerufen, die bei den verfahrensmässig nicht zu vermeidenden Temperaturen, Feuchtigkeitsverhältnissen und der mechanischen Beanspruchung diese unangenehmen Eingenschaften zeigen.

Es wurde nun gefunden, dass der Feststoff aus der Salinomycin-Kulturbrühe verbackungsfrei dadurch isoliert werden kann, dass man bei der Fermentation einen nicht über 3,5% liegenden Gehalt an extrahierbaren Fetten und fettänlichen Substanzen erreicht und während der Zerstäubungstrocknung ein physiologisch verträglisches, feinpulvriges Material, das als Antibackmittel bezeichnet werden soll, zugibt.

Die einzelnen Schritte des erfindungsgemässen Verfahrens lassen sich beispielsweise folgendermassen beschreiben.

Die fermentative Herstellung von Salinomycin wird in an sich bekannter Weise (vgl. z.B. Y. Miazaki et al., J. Antibiotics 27, S. 814-821 (1974); H. Kinashi et al., Tetrahedron letters 49, S. 4955-4958 (1973) und H. Kinaski et al., Agr. Biol. Chem. 40 S. 1625-1632 (1976)) durchgeführt, wobei als Kohlenstoffquelle und Antischaummittel hauptsächlich Fette unterschiedlicher Provenienz, wie z.B. Soja-Oel, Sesam-Oel, Raps-Oel, Saflor-Oel, Oliven Oel, Dorsch-Oel, Methyloleat, Methylmyristat und Methyllinoleat dienen.

Die Fermentation wird so lange geführt, dass zum Zeitpunkt der Aberntung der Anteil dieser Fette oder daraus durch Abbau entstandene fettähntliche Produkte, wie z.B. Laurinsäure, Myristinsäure, Palmitinsäure, Stearinsäure, Arachnoinsäure, Oelsäure, Linolsäure und Palmitoleinsäure, die durch Extraktion mit nicht mit Wasser mischbaren organischen Lösungsmitteln, wie z.B. Methylenchlorid, Benzin, Benzol, Chloroform, Schwefelkohlenstoff, Tetrachlorkohlenstoff, Tetralin oder Trichloräthylen, zusammen mit Salinomycin extrahiert und in üblicher Weise (vgl. z.B. Römpp Chemie-Lexikon, 7. Aufl., 2. Band, S. 1072, Stichwort «Extraktion»; Deutsche Lebensmittel-Rundschau 69 (1973), S. 470-472) bestimmt werden können, nicht über 3,5%, vorzugweise unter 2% der Kulturbrühe liegt, wobei in diesem Wert der Gehalt an Salinomycin nicht mehr enthalten ist. Die Erreichung dieses Wertes wird durch laufende Probeentnahme überprüft.

Um die Zerstäubungstrocknung wirtschaftlicher zu gestalten, kann die Kulturbrühe in einem Verdampfer in bekannter Weise voreingedickt werden, beispielsweise von einem Feststoffgehalt von 13 bis 20% auf einen solchen von 30 bis 50%, vorzugsweise 40%. Entscheidend für den Umfang einer Voreindickung ist die Pumpfähigkeit des Produktes. Förderung und Zerstäubung müssen noch gut möglich sein.

Die Zerstäubungstrocknung wird auf an sich bekannte Weise in einem Trockenturm (zylindrischer Turm mit konischem Austrag), wie zum Beispiel einem Scheibenzerstäubungstrockner oder Düsentrocker durchgeführt. Verwendung finden kann z.B. ein Scheibenzerstäubungsturm von 2,2 m Durchmesser mit einer Zylinderlänge von 1,2 m und angesetztem 60°-Konus oder ein Düsenzerstäubungsturm mit einem Durchmesser von 1,6 m, einem 4 m langen Zylinder und 60°-Konus.

Die Eintrittstemperatur der Luft kann zwischen 140 und 220° C, vorzugweise zwischen 190 und 210° C, die Austrittstemperatur zwischen 80 und 100° C, vorzugsweise zwischen 85 und 95° C liegen.

Ein optimales Trocknungsergebnis wird erzielt, wenn die relative Luftfeuchtigkeit bei Austritt aus dem Trockenturm 8 bis 20%, vorzugweise 10 bis 15% beträgt.

In Gegenwart von Zündquellen kann zur Vermeidung von Staubexplosionen auch ein Gasstrom mit einem gegenüber dem normalen Gehalt reduzierten Sauerstoffgehalt von beispielsweise 8 bis 15%, vorzugsweise unter 14% eingesetzt werden.

Als erfindungsgemäss hinzugefügtes Antibackmittel kann jedes physiologisch verträgliche feinpulvrige Material dienen, das geeignet ist, die plastischen Eingenschaften des Feststoffes aus der Kulturbrühe — vermutlich durch einen Verdünnungs- und Umhüllunggseffekt der sprühgetrockneten Partikel — so weit zurückzudrängen, dass eine Verklumpung und Verklebung, die insbesondere unter den erforderlichen Temperatur- und Druckbedingungen auftritt, nicht mehr zu beobachten ist.

Vorzugsweise kommen in Betracht fein verteilte Calciumcarbonate und Kieselsäuren natürlicher oder synthetischer Herkunft, wie z.B. Kreide, gefällte Kieselsäure, Diatomeenerde, auch Talkum und Kaolin sind geeignet, wobei die Antibackmittel sowohl allein als auch in Mischungen miteinander eingesetzt werden können. Erfindungsgemäss hat sich insbesondere ein Zusatz von Kieselgur (wie z.B. Diamol GMR) als günstig erwiesen.

Die Zugabe des Antibackmittels kann beispielsweise so erfolgen, dass es über eine Dosiereinrichtung (z.B. eine Dosierschnecke, Vibrationsrinne oder ein Dosierband) dem in üblicher Weise im Gleichstrom mit der Kulturbrühe von oben in den Trockner eingeleiteten Hauptluftstrom zugegeben wird, vorzugsweise jedoch einem Nebenluftstrom, der so geführt wird, dass die Antibackpartikel auf die bereits weitgehend getrockneten Teile des Feststoffs der Kulturbrühe auftreffen. Die Zudosierung erfolgt im freien Fall oder mit einem geeigneten Zerstäuber, z.B. einer Zweistoffdüse an einer oder mehreren, vorzugsweise an drei Stellen. Bei einem Düsenzerstäubungsturm wird der Nebenluftstrom vorzugsweise nahezu senkrecht zur Trocknerluft geführt. Das erfindungsgemässe Verfahren kann im Prinzip auch mit einem im Gegenstrom arbeitenden Trockner durchgeführt werden.

In Abhängigkeit von den jeweiligen Produkteigenschaften (Anbacken insbesondere bei Belastungen mit Druck und Temperatur) können beispielsweise 1 bis 25%, vorzugsweise 4-10% (bezogen auf eine eingesetzte Kulturbrühe, die im allgemeinen 10 bis 25% Feststoff enthält) Antibackmittel zugegeben werden, die über den gesamten Zeitraum der Trocknung gleichmässig zudosiert werden. Auch durch die Art des Antibackmittels kann die Zugabemenge beeinflusst werden. So können beispielsweise bei Verwendung von Aerosil bereits zwischen 1 und 2% liegende Mengen ausreichen, bei Verwendung von Kieselgur 4 bis 5%, um einen sprühgetrockneten Feststoff, der 15 bis 25% Salinomycin enthält, zu erhalten.

Auch eine Steigung der Antibackmittelmenge auf weit über 25% liegende Werte ist ohne weiteres möglich, wobei jedoch in der Regel keine wesentliche Verbesserung des Produktes erreicht wird.

Lassen sich bei der Fermentation die oben angegebenen niedrigen, möglichst nicht über 3%, vorzugsweise unter 2% liegende Werte an extrahierbaren Fetten oder fettähnlichen Produkten nicht oder nur schwer erreichen und neigt die Kulturbrühe damit besonders stark zum Verklumpen, kann es zweckmässig sein, zumindest einen Teil der gesamten Antibackmittelmenge bereits vor dem Zerstäubungstrocknen in die Kulturbrühe einzurühren. Dies kann zu jedem Zeitpunkt vor der Zerstäubungstrocknung erfolgen. So können z.B. zwischen 2 und 10%, vorzugsweise zwischen 4 und 5% (bezogen auf die Kulturbrühe) liegende Mengen eingerührt werden, wobei auch hier der 10%-Wert ohne weiteres überschritten werden kann. Das Einrühren erfolgt mit geeigneten Eintragsorganen.

Die Austragung des getrockneten Feststoffs erfolgt in bekannter Weise, beispielsweise über eine pneumatische Schleuse und pneumatische Absaugung.

Zweckmässig kann es sein, zur Erreichung einer möglichst homogenen Einspeisung in den Trocknerturm gegebenenfalls vorhandene Agglomerate, die gelegentlich nach Zugabe von Inertmaterial oder bei nicht ganz frisch fermentierten Kulturbrühen auftreten können, vor der Zerstäubungstrocknung zu zerkleinern, was beispielsweise durch Nassvermahlung mit Kolloidmühlen in bekannter Weise erfolgen kann.

Das Ergebnis des erfindungsgemässen Verfahrens war überraschend, nachdem es in zahlreichen Vorversuchen nicht gelungen war, die Isolierung des Feststoffes weitgehend verhindernden Klumpenbildung allein dadurch zu beseitigen, dass der Gehalt an Fetten oder fetthaltigen Substanzen während der Fermentation stark herabgesetzt oder das Antibackmittel nur zu der Kulturbrühe hinzugefügt wurde. Erst durch die erfindungsgemässen Massnahmen war es möglichst, ein als Futterzusatz sehr gut geeignetes Produkt auf einfache Weise und mit niedrigen Herstellungskosten zu erhalten.

Die Zusammensetzung des erfindungsgemäss anfallenden, getrockneten Feststoffs aus der Salinomycin-Kulturbrühe kann in Abhängigkeit von der Zusammensetzung der Kulturbrühe in gewissen Bereichen schwanken. In der Regel wird der erfindungsgemäss gewonnene, salinomycinhaltige Feststoff einen zwischen 5 und 60%, vorzugsweise zwischen 8 und 25% liegenden Gehalt an Antibackmittel und einen Salinomycingehalt von 15 bis 30%, vorzugsweise zwischen 18 und 25% aufweisen.

*Beispiel 1*

Nach einer Fermentation mit vollständigem Fettabbau lag eine Kulturbrühe mit einem Feststoffgehalt von 20% vor. Der Mikroorganismus wurde durch Zugabe von 1,0% Formalin (35%-ige) wässrige Lösung), bezogen auf die Kulturbrühe, abgetötet und der pH-Wert der Kulturbrühe mit 30%iger Natronlauge auf pH 9,5 eingestellt.

Die Aufgabe auf den Scheibenzerstäubungsturm mit einem Durchmesser von 2,2 m, einer Zylinderlänge von 1 m, einem angesetzten Konus von 60° und einer Scheibe von 120 mm Durchmesser mit 24 000 UpM, erfolgte mit einer Exzenterschneckenpumpe.

Die Eingangstemperatur des Trockners betrug 200°C, zugeführt wurden 1200 kg Trocknungsgas pro Stunde mit einem unterhalb von 14% liegendem Sauerstoff-Gehalt. Bei einer Einspeisung von 85 kg Kulturbrühe pro Stunde betrug die Trockner- Ausgangstemperatur 85°C.

Über eine Dosierschnecke wurden 5% Kieselgur, bezogen auf die Kulturbrühe, über einen Trichter in freiem Fall in den Turm zudosiert. Die Austragung aus den Zyklonen erfolgte durch Vortex-Schleusen.

95% des eingesetzten Feststoffes aus der Kulturbrühe wurden so in Form eines rieselfähigen Materials erhalten. Der Produktionsstamm war im Produkt nicht nachweisbar.

*Beispiel 2*

Nach einer Fermentation mit nicht ganz vollständigem Fettabbau (3,5% Restfett) wurden die Mikroorganismen der Kulturbrühe, die 18% Feststoff enthielt, durch Zugabe von 1% Formalin (35%ige wässrige Formalinlösung), bezogen auf die Kulturbrühe, abgetötet und der pH-Wert der Kulturbrühe mit 30%iger Natronlauge auf pH 10 eingestellt.

Um das stärker zum Verklumpen und Verbacken neigende Produkt pump- und sprühfähig zu machen, wurden in die Kulturbrühe 5% Kieselgur (Diamol GM[R]) eingerührt und schon vorhandene Verklumpungen vor dem Sprühtrocknen durch eine Kolloidmühle zerkleinert.

Die Aufgabe des so vorbereiteten Materials auf einen Zweistoffdüsentrockner erfolgte mit einer Exzenterschneckenpumpe. Der Trockner hatte einen Durchmesser von 1,2 m mit einem 4 m langen Zylinder und einem angesetzten 60°-Konus.

Die Eingangstemperatur betrug im Trockner 200°C. Zugeführt wurden 900 kg Trocknungsgas pro Stunde mit einer unter 14% liegenden Sauerstoffkonzentration. Bei der Einspeisung von 45 kg Kulturbrühe pro Stunde betrug die Trockner-Ausgangstemperatur 85°C.

Über eine Dosierschnecke wurde senkrecht zum Trocknungsgasstrom ebenfalls über eine Zweistoffdüse 5% Kieselgur (Diamol GM^R) in den Trockner-Turm gebracht.

92% des eingesetzten Feststoffes aus der Kulturbrühe fielen so in Form eines rieselförmigen Produkts an. Versuche, den Produktionsstamm aus dem Sprühpulver zu isolieren, blieben erfolglos.

*Beispiel 3*

Kulturbrühe, wie in Beispiel 1 angegeben, wurde vor der Eingabe in den Trockenturm in einem Dünnschichtverdampfer eingeengt. Die Feststoffmasse betrug danach 40%. Die Trocknung erfolgte wie im Beispiel 1 beschrieben.

*Beispiel 4*

Eine wie im Beispiel 2 angegebene Kulturbrühe wurde in der dort beschriebenen Weise getrocknet, wobei anstelle von Kieselgur als Antibackmittel 15% Kreide (bezogen auf die Kulturbrühe) verwendet wurden, wovon 5% in die Kulturbrühe eingerührt und 10% dem Trocknungsgas zugegeben wurden.

## Patentansprüche

1. Verfahren zur Isolierung des Feststoffs aus der Salinomycin-Kulturbrühe, dadurch gekennzeichnet, dass bei der Fermentation ein nicht über 3,5% liegender Gehalt an extrahierbaren Fetten und fettähnlichen Substanzen erreicht und während der Zerstäubungstrocknung ein physiologisch verträgliches Antibackmittel zugegeben wird.

2. Verfahren nach Anspruch 1, dadurch gekennzeichnet, dass der zu erreichende Gehalt an extrahierbaren Fetten und fettähnlichen Substanzen unterhalb 2%, bezogen auf die Kulturbrühe, liegt.

3. Verfahren nach Anspruch 1, dadurch gekennzeichnet, dass das Antibackmittel ein feinpulvriges Material darstellt, das die plastischen Eigenschaften des Feststoffs aus der Salinomycin-Kulturbrühe so weit zurückdrängt, dass ein Verbacken nicht mehr auftritt.

4. Verfahren nach Anspruch 3, dadurch gekennzeichnet, dass als Antibackmittel Calciumcarbonate oder Kieselsäuren natürlicher oder synthetischer Herkunft verwendet werden.

5. Verfahren nach Anspruch 1, dadurch gekennzeichnet, dass ein Teil der gesamten Antibackmittelmenge der Kulturbrühe bereits vor der Zerstäubungstrocknung zugegeben wird.

6. Verfahren nach Anspruch 5, dadurch gekennzeichnet, dass eine Zugabe von Antibackmittel vor der Zerstäubungstrocknung dann erfolgt, wenn bei der Fermentation nicht eine Senkung des Gehaltes an extrahierbaren Fetten und fettähnlichen Substanzen auf mindestens 3% erreicht werden kann.

7. Feststoff aus der Salinomycin-Kulturbrühe, gekennzeichnet durch einen Gehalt an physiologisch verträglichem Antibackmittel.

## Revendications

1. Procédé pour l'isolement de la substance solide à partir du bouillon de culture de la salinomycine, caractérisé en ce que par fermentation on obtient une teneur au plus égale à 3,5% en graisses et substances analogues à de la graisse, extractibles et que pendant le séchage par atomisation on ajoute un agent anti-agglomération physiologiquement acceptable.

2. Procédé selon la revendication 1, caractérisé en ce que la teneur à atteindre en graisses et en substances analogues à de la graisse, extractibles, est inférieure à 2% par rapport au bouillon de culture.

3. Procédé selon la revendication 1, caractérisé en ce que l'agent anti-agglomération est un matériau finement pulvérisé qui réprime les propriétés plastiques de la substance solide provenant du bouillon de culture de la salinomycine, de telle sorte qu'il ne peut plus se produire d'agglomération.

4. Procédé selon la revendication 3, caractérisé en ce que comme agent anti-agglomération, on utilise le carbonate de calcium ou des silices d'origine naturelle ou synthétique.

5. Procédé selon la revendication 1, caractérisé en ce qu'une partie de la quantité totale d'agent anti-agglomération est ajoutée au bouillon au culture avant le séchage par atomisation.

6. Procédé selon la revendication 5, caractérisé en ce qu'une addition d'un agent anti-agglomération avant le séchage par atomisation se fait lorsque par fermentation on ne peut pas obtenir une diminution de la teneur en graisses et en substances analogues à de la graisse, extractibles, à 3% au minimum.

7. Substance solide provenant du bouillon de culture de la salinomycine, caractérisé par une teneur en agent anti-agglomération physiologiquement acceptable.

## Claims

1. Process for the isolation of the solid matter from the salinomycin culture broth, wherein in the fermentation a content of extractable fats and fat-like substances not exceeding 3,5% is reached, and a physiologically acceptable anti-agglomeration agent is added during the spray drying operation.

2. Process as claimed in claim 1, wherein the content of extractable fats and fat-like substances to be reached is less than 2%, calculated on the culture broth.

3. Process as claimed in claim 1, wherein the anti-agglomeration agent represents a finely powdered material which supresses the plastic properties of the solids from the salinomycin culture broth to an extent that agglomeration does no longer occur.

4. Process as claimed in claim 3, which comprises using as anti-agglomeration agents calcium carbonates or silicic acids of natural or synthetic origin.

5. Process as claimed in claim 1, which comprises adding part of the total amount of anti-agglomeration agent to the culture broth already prior to the spray drying.

6. Process as claimed in claim 5, wherein an anti-agglomeration agent is added prior to the spray drying in those cases where in the fermentation stage the content of extractable fats and fat-like substances could not be reduced to a value of at least 3%.

7. Solid matter from the salinomycin culture broth, which contains a physiologically acceptable anti-agglomeration agent.